# EUROPEAN PATENT SPECIFICATION

(11) **EP 2 956 069 B1**
(45) Date of publication and mention of the grant of the patent: **02.11.2016**
(21) Application number: 14704634.6
(22) Date of filing: 05.02.2014
(51) Int. Cl.: A61B 17/15

(54) **A GUIDE FOR LOCATING A CUTTING BLOCK ON A PATIENT'S FEMUR**
FÜHRUNG ZUR LOKALISIERUNG EINES SCHNEIDBLOCKS AUF EINEM OBERSCHENKELKNOCHEN EINES PATIENTEN
GUIDE POUR LOCALISER UN BLOC DE COUPE SUR LE FÉMUR D'UN PATIENT

(30) Priority: 18.02.2013 GB 201302782
(43) Date of publication of application: 23.12.2015
(73) Proprietor: DePuy (Ireland), Ringaskiddy (IE)
(72) Inventor: LESLIE, Ian, Leeds West Yorkshire LS11 8PA (GB); REEVE, Michael, Leeds West Yorkshire LS11 8PA (GB)
(74) Representative: Belcher, Simon James
(86) International application number: PCT/GB2014/050329
(87) International publication number: WO 2014/125253

(56) References cited:
- WO-A1-2011/141723
- US-A- 5 860 980
- US-A1- 2011 046 685
- US-B1- 7 488 324

## Description

This invention relates to a guide for locating a cutting block on a patient's femur in a knee replacement procedure, and to kits which include the guide and methods of using the guide.

Factors on which the success of a knee replacement surgical procedure depends include selection of appropriate implant components and preparing the patient's bone so that the implants are positioned appropriately. Instruments are used widely to measure the size of the patient's bones, and to identify the proper locations on the bones where the bones should be cut to receive the implant components.

Preparing the femur to receive the chosen size of femoral component requires the surgeon to perform resection cuts on the femur. It is common for the first cut that is performed to be the distal cut. This is frequently performed before the choice of the size of the femoral component has been finalised. The positions of the anterior and posterior cuts are frequently controlled during the cutting steps using one or more cutting blocks. It is common to use one or more instruments to position a cutting block, for example relative to landmark features on the patient's bones in the vicinity of the patient's joint or other anatomical features.

A commonly employed approach to positioning a cutting block that is used to cut the posterior condyles involves use of anatomical features on the femur, for example involving locating the anterior-posterior axis which extends between the trochlear and intercondylar grooves, and the medial-lateral axis. The surgeon must then estimate an appropriate angular offset between the resection plane and the medial-lateral axis. This depends on the amount of rotation in the patient's joint, which can be seen in the angle between the posterior cut and the medial-lateral axis, about the axis of the femur.

The degree of femoral rotation that is set by the position of the cutting block will affect the relative tension in the soft tissue which spans the knee joint, in the medial and lateral compartments respectively. A surgeon might assess the soft tissue tension after positioning
a cutting block on the distal face of the femur with reference to anatomical features on the femur. However, it can be difficult then to adjust the position of the cutting block.

WO-A-2011/141723 discloses a femoral sizing guide comprising a foot component and an extension piece that are rotatably coupled together. A lever, pivotally coupled to the extension piece, functions as a locking mechanism and locks the extension piece in a predetermined rotational position relative to the foot component. The foot component includes foot locator surfaces arranged to bear against respective posterior condylar surfaces. The extension piece includes first and second pairs of guide holes for determining the axes of alignment holes in either a "posterior up" or "anterior down" approach to positioning of a femoral prosthesis.

Another approach to positioning a cutting block involves use of the proximal surface of the tibia as a reference, with the medial and lateral soft tissue being tensioned as appropriate. The tibia can be resected prior to it being used as a reference. The surface of the tibia which is used as a reference can be the surface of the bone itself, or it can be the surface which is provided by an instrument or an implant component which is positioned on the tibia after it has been resected.

Different instruments have to be provided in order for a surgeon to be able to choose between locating a cutting block relative to anatomical features on the femur, and locating it relative to the proximal surface of the tibia with the joint soft tissue placed in tension.

The present invention provides a guide for locating a cutting block on a patient's femur, in which an anterior body part has a pair of locator holes formed in it for locating fastener pins for a cutting block, and has a ledge extending from the face which is opposite to the face which contacts the distal face of the femur, the ledge defining a plane which is coincident with the intended posterior condyle resection plane.

The present invention provides a guide as defined in claim 1 for locating a plane on which the posterior portions of the femoral condyles are to be resected in a knee replacement procedure and for locating a cutting block on a patient's femur.

When the posterior body part has been positioned against the distal face of the femur, with the posteriorly extending tab portion fitted against the posterior condyles, the anterior body part can be rotated about the rotation axis (which extends approximately perpendicular to the distal face of the femur) until its position is such that a cutting block, which is fitted to the femur using fastener pins which are located using the locator holes, defines a plane on which the posterior condyles are resected. The plane which is defined by the cutting block (when fitted on to the fastener pins) is then arranged at a rotation angle relative to the plane defined by the patient's posterior condyles as selected by the surgeon using the guide of the invention to suit the anatomy of the patient. The lock can be used to prevent rotation of the anterior body part from that position.

The selection of the rotation position of the anterior body portion relative to the posterior body position can be made by the surgeon with reference to anatomical features on the femur such as, for example, one or more of the trochlear and intercondylar grooves and/or the medial/lateral axis. The anterior body portion can include reference indicia (for example reference marks or notches or ribs) for alignment with these and/or other anatomical features.

The selection of the rotation position can be made with reference to the proximal surface of the tibia, with the medial and lateral soft tissue which extends between the femur and the tibia being tensioned as appropriate. Techniques for placing the soft tissue in tension are well known, for example using distractor instruments such as the instrument which is disclosed in WO-A-2014/006360, or the instrument which is disclosed in WO-A-2007/036699. The selection of the rotation position will generally involve use of a spacer block which is positioned between the ledge and the tibia, in contact with the ledge and the tibia. The thickness of the spacer block which fits between the ledge and the tibia when the soft tissue is appropriately tensioned provides an indication to the surgeon of the thickness of the implant components which are required to be located between the implanted femoral component and the patient's tibia. Such implant components will generally include a tibial component and a bearing component. The surgeon might choose a bearing component having a thickness which is appropriate having regard to the thickness of the spacer block.

The ledge can provide the surgeon with a visual indication of the location of the plane of the resection cut for the posterior condyles. It can be preferred that the ledge is coincident with the intended resection plane for the posterior condyles.

The angle between the anterior and posterior body parts is considered to be zero when the plane which is defined by the posterior facing surface of the ledge and the plane which is defined by the posterior condyles of the patient's knee are parallel to one another.

The distance between the plane which is defined by the posterior facing surface of the ledge and the plane which is defined by the posterior condyles of the patient's knee is determined by the distance between the bone engaging surface of the posterior condyles of the femoral component and the opposite portion of the articulating surface. The distance between the two planes will frequently be between 5 and 12 mm, for example about 8 mm.

Preferably, the surface of the ledge which faces posteriorly is planar. Preferably, the plane defined by the surface of the ledge is arranged so that it extends approximately perpendicular to the distal end face of the resected femur when the guide is in position against that end face. It can be preferred that the plane defined by the surface of the ledge is arranged so that it extends approximately parallel to the surface of the posteriorly extending tab portion or portions on the posterior body part.

The depth of the ledge, at or close to the middle of the ledge along the medial-lateral axis, measured in a direction which is parallel to the femoral axis, is preferably at least about 5 mm, more preferably at least about 10 mm, for example at least about 15 mm. Preferably, the width of the ledge satisfies at least one of these limitations along a portion of its width (measured in a direction which is parallel to the medial-lateral axis) measuring at least about 1 cm, more preferably at least about 2 cm, especially at least about 3 cm, for example at least about 4 cm.

Preferably, the width of the ledge measured in a direction which is parallel to the medial-lateral axis is at least about 2 cm, more preferably at least about 3 cm, especially at least about 4 cm.

The anterior body part can have pin holes for guiding pins which can be used to fasten cutting blocks in two different positions on the femur. The instrument can therefore be used to locate the pins on the femur. The pins remain in place in the positions in which they have been located using the instrument after the instrument has been removed. They can then be received in bores in a cutting block so as to locate the cutting block appropriately on the face of the femur for use in subsequent cutting steps.

The anterior body part can be used to fasten a cutting block in a first position which is suitable for a procedure which is referred to as "anterior down" in which the first cut that is made using the cutting block is the anterior cut. The anterior body part can be used to fasten a cutting block in a second position which is suitable for a procedure which is referred to as "posterior up" in which the first cut that is made using the cutting block is the posterior cut. The anterior body part can include one, two or more (especially two) pin holes for fastening a cutting block in each position on the femur (for example the positions for the "anterior down" and "posterior up" procedures). The pin hole or holes for fastening a cutting block on the anterior body part of a guide for use in an "anterior down" procedure will frequently be located anteriorly of the pin hole or holes which are used to fasten a cutting block for use in a "posterior up" procedure.

Preferably, the guide includes a stylus for engaging the anterior cortex. Preferably, the stylus is mounted on the anterior body part. Preferably, the anterior body part includes a mounting fixture for mounting the stylus. Preferably, the mounting fixture allows the stylus to be moved relative to the anterior body part so as to change the position on the anterior face of the femur at which the tip of the stylus contacts the femur.

Preferably, the anterior body part comprises a first portion which is connected to the posterior body part, and a second portion on which the stylus mounted, the connection between the first and second portions allowing the second portion to move relative to the first portion, to vary the distance between the posteriorly extending tab portion on the posterior body part and the stylus. One of the first and second portions, especially the second portion, can have a pair of arms which slide in a pair of corresponding channels in the other of the first and second portions.

The stylus can be used to measure the size of the femur, by positioning the posterior body part of the guide against the distal face of the femur with the posteriorly extending tab portion fitted against the posterior condyles, and moving the second portion of the anterior body part relative to the first portion and to the posterior body part until the stylus contacts the anterior face of the femur. In this way, the guide provides an indication of the distance from the posterior face of the condyles to the anterior face of the femur, measured in a direction which is parallel to the anterior-posterior axis. The guide can include a scale which can provide an indication to the surgeon of the size of the femur. For example, the scale can indicate the optimum size of femoral component that is appropriate having regard to the size of the femur. The scale on the instrument can have markings on it which correspond to respective femoral components which have different sizes. For example, the scale might have markings on it corresponding to ten different sizes of femoral components, having different measurements between the posterior condyles and the anterior cortex. The anterior-posterior distance can differ by about 5 mm between sizes of femoral components. The guide can include a lock by which the second portion of the anterior body part can be locked against movement relative to the first portion thereof. Preferably the lock allows the first and second portions to be moved between a continuous range of positions. The lock can operate with a rod mounted on one of the portions which is received in a bore on the other of the portions. Operating the lock causes the bore to be compressed to grip the rod so that further movement of the rod within the bore is prevented. Mechanisms to cause a rod to be gripped in this way are well known. The rod can be one of a pair of arms on one of the first and second portions which are received in respective channels in the other of the first and second portions.

The stylus can be mounted on the second portion of the anterior body part in such a way that it can slide relative to the anterior body part. The stylus can define a track. The stylus can engage a locator on the anterior body part. The locator can slide relative to the track so that the stylus can slide relative to the anterior body part. Such movement of the stylus relative to the anterior body part allows the distance between the anterior body part and the tip of the stylus to be adjusted so that the tip of the stylus engages the anterior cortex of the femur at the position beyond the sulcus which is considered appropriate by the surgeon. Use of a stylus in this way to measure the size of a femur is commonplace in knee replacement surgical procedures.

It will generally be preferred that the anterior body part rotates relative to the posterior body part around a single axis. The axis can be located on or reasonably close to the medial-lateral centerline of the femur. The distance between the posteriorly extending tab portion and the axis about which the anterior body part rotates relative to the posterior body part can be approximately equal to the distance between the posterior condyles and the intramedullary axis of the femur in the case of a femur whose size is such that it will take a femoral component in the middle of the range of size of femoral component. In the case of a larger femur, the distance between the posteriorly extending tab and the axis will be less than the distance between the posterior condyles and the intramedullary axis of the femur. In the case of a smaller femur, the distance between the posteriorly extending tab and the axis will be greater than the distance between the posterior condyles and the intramedullary axis of the femur.

Optionally, the posterior body part can include at least one feature which can be used to fix the posterior body part against movement relative to the femur. The feature can be in the form of one or more openings (for example two openings) in which a fastener can be inserted to extend into the face of the femur. The fastener might be a pin or a screw. The fastener can be used to stop movement of the guide in the plane that is defined by the distal face of the femur. It can be preferred that the posterior body part has two openings formed in it so that it is fastened against rotation relative to the distal face of the femur and against sliding across that face.

Optionally, the lock which is used to lock the anterior body part and the posterior body part against relative rotation can be switched between a locked position in which the anterior body part and the posterior body part are locked against relative rotation and an unlocked position in which the anterior body part is able to rotate relative to the posterior body part. The lock can be biassed towards the locked position. This can provide security against unwanted movement of the anterior body part relative to the posterior body part.

Optionally, the lock which is used to lock the anterior body part and the posterior body part against relative rotation can define a plurality of discrete angular orientations of the anterior body part relative to the posterior body part. For example, one of the body parts can bear a plurality of indentations and the other body part can bear a ridged pawl which can engage a selected one or more of the indentations to fasten the body parts in a desired angular orientation relative to one another. The plurality of indentations can be provided in a fixed rack and the pawl can be moved between a locked position in which it engages a selected one or more of the indentations and an unlocked position in which it is disengaged from the indentations. The pawl can be biassed towards the locked position. The pawl can be mounted on one of the body parts so as to pivot about an axis between the locked and unlocked positions. A lever can be provided on the pawl to move the pawl between the locked and unlocked positions. It can be preferred for the plurality of indentations to be provided on the posterior body part and for the pawl to be provided on the anterior body part. The plurality of indentations can be in the form of a toothed rack.

Preferably, the angle between the anterior and posterior body parts can be varied in a range of at least about ±2°, more preferably at least about ±3°, for example at least about ±4°. The angle between the anterior and posterior body parts will not normally be variable through a range which is larger than ±7°. When the lock locks the anterior body part and the posterior body part against relative rotation in a plurality of discrete angular orientations, the difference between the angles in consecutive orientations might be between about 0.3° and about 1.5°, for example between about 0.5° and about 1.0°.

The invention also provides a kit for use in locating a cutting block on a patient's femur in a knee replacement procedure, the cutting block being used to locate the resection plane for the posterior condyles, which comprises a guide as discussed above and a spacer block for fitting between the ledge and the proximal surface of the tibia.

The spacer block can be positioned between the ledge and the proximal surface of the tibia to provide the surgeon with an indication of the tension in soft tissue which extends between the femur and the tibia on the basis of the position of the posterior condyle resection plane being as indicated by the position of the ledge, and on the basis of the knee prosthesis components having a size (that is, a thickness) as represented by the spacer block. The kit can include a plurality of spacer blocks which have different thicknesses. The different thicknesses of spacer blocks can represent different sizes of bearing components which are positioned between the tibial and femoral components of a knee prosthesis.

The invention also provides a kit for use in locating a cutting block on a patient's femur in a knee replacement procedure, which comprises a guide as discussed above, and a cutting block for locating the resection plane for the posterior condyles, the cutting block having a pair of fixation holes formed in it for receiving fastener pins for fastening the cutting block to the femur, and having a guide surface for guiding a cutting instrument to cut the posterior condyles, the relative location of the guide surface and the fixation holes on the cutting block being the same as the relative location of the location holes and the posterior facing surface of the ledge on the anterior body part.

The instrument can be made from materials which are conventionally used in the manufacture of surgical instruments, including in particular certain stainless steels.

Embodiments of the invention will now be described by way of example with reference to the accompanying drawings, in which:
Figure 1 is an isometric view of a guide for locating a cutting block on a patient's femur in a knee replacement procedure, showing the proximal face with the anterior and posterior body parts separated
Figure 2 is an isometric view of the guide which is shown in Figure 1, showing the anterior face with the anterior and posterior body parts assembled.
Figure 3 is an exploded view from below of the guide which is shown in Figure 1.
Figure 4 is a side view of a patient's femur and tibia, with the guide in place in contact with the posterior and distal surfaces of the femoral condyles.

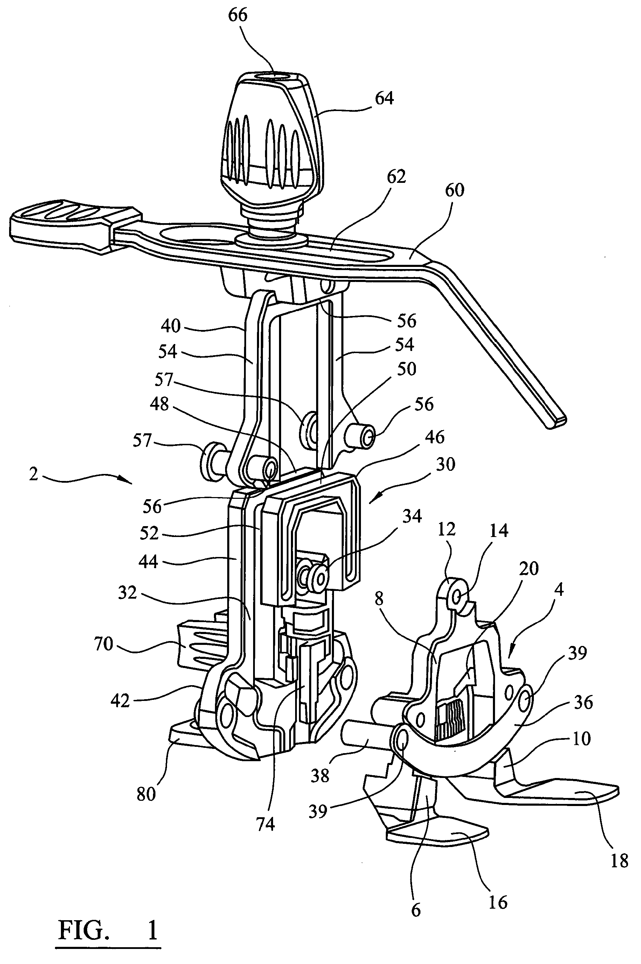

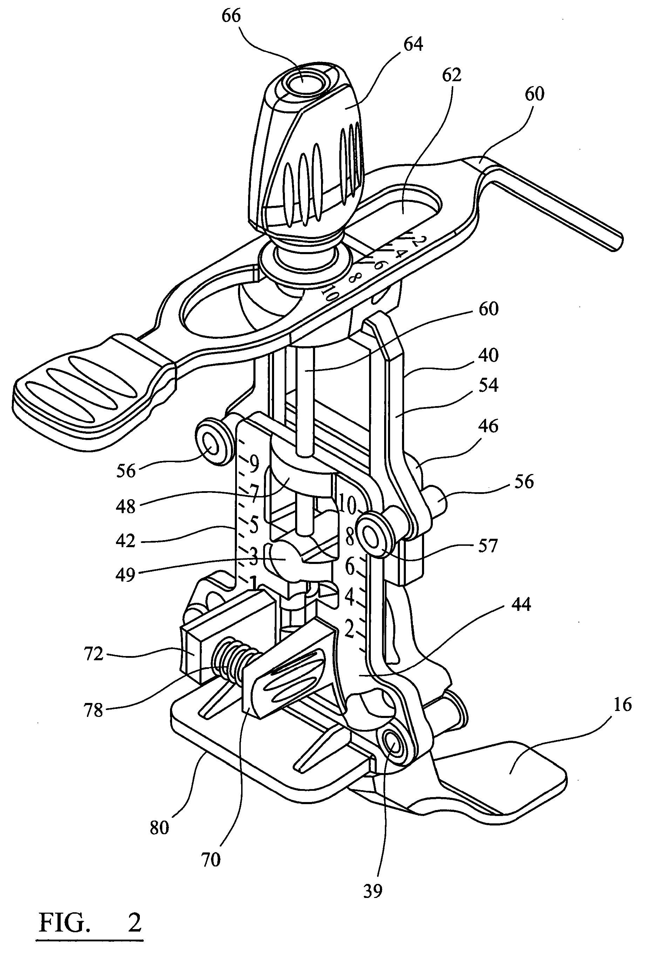

Referring to the drawings, Figures 1 to 3 show a guide 2 for locating a cutting block on a patient's femur where the cutting block is intended to control the action of a saw blade which is used to resect the posterior portion of the femoral condyles. The guide has a posterior body part 4 having a proximal face 6 for positioning against the distal face of the femur. The posterior body part has first and second limbs 8, 10 which are connected to one another at an apex 12. A through-hole 14 is provided in the posterior body part at the apex 12. First and second posteriorly extending tab portions 16, 18 extend from the first and second limbs respectively. The tab portions can be fitted against the posterior condyles.

A toothed plate 20 is provided on the posterior body part, extending between the first and second limbs. The teeth are provided on the face of plate which faces posteriorly, that is in the same direction as that in which the tab portions extend from the first and second limbs. The teeth are arranged in an array with each tooth extending approximately radially. The array is centred on the centre of the through-hole.

The guide 2 includes an anterior body part 30 having a proximal face 32 for positioning against the distal face of the femur. The anterior body part has a spigot 34 which can be received in the through-hole 14 at the apex 12 on the posterior body part to fasten the anterior body part to the posterior body part such that one can rotate relative to the other about the axis defined by the spigot and the through-hole.

The guide includes an arcuate plate 36 which is fastened to the anterior body part at the opposite ends of the arcuate plate through a pair of spacer lugs 38. The posterior body part is positioned between the anterior body part and the arcuate plate. The arcuate plate helps to prevent movement of the posterior body part relate to the anterior body part other than rotation about the axis defined by the spigot 34. The spacer lugs are hollow along their length, defining a pair of posterior pin fixation bores 39.

The anterior body part has a frame which has an upper portion 40 and a lower portion 42. The lower portion 42 is narrower than the upper portion, and carries the posterior body part of the guide. The lower portion has a front plate 44 and a back plate 46. Each of the front and back plates has a pair of spaced apart limbs. The limbs of the front plate are connected by upper and lower front plate bridges 48, 49. The spigot 34 which is received in the through-hole 14 on the posterior body part is mounted on the lower front plate bridge. The limbs of the back plate are connected by a back plate bridge 50. The lower portion is made (for example moulded or cast in one piece) so that the limbs of the back plate are spaced apart from the limbs of the front plate. In this way, the limbs define a pair of spaced apart channels 52 between them, one channel on each side of the lower portion.

The limbs of the front plate of the lower portion of the anterior body part have scale markings on them which will provide information to the surgeon as to the size of the patient's femur. The scale markings correspond to different sizes of femoral components. The markings for odd numbered sizes are provided on the left limb and the markings for even numbered sizes are provided on the right limb.

The upper portion 40 of the anterior body part has a pair of spaced apart limbs 54 which are connected by an upper portion bridge 56. Each of the limbs on the upper portion is sized to be a snug sliding fit in a respective one of the channels which are defined between the front and back plates of the lower portion 42 of the anterior body part. Each of the limbs of the upper portion is flared outwardly towards its free end and has an anterior pin fixation bore 56 extending through it. A rim 57 surrounds each of the anterior pin fixation bores 56. A horizontal groove is provided in each of the rims, which is read against the scale markings on the limbs of the front plate of the lower portion of the anterior body part.

A guide shaft 60 extends from the lower front plate bridge 48 of the lower portion of the anterior body part, through a bore in the upper plate bridge 49 and a bore in the upper portion bridge 56. The guide shaft guides the sliding movement of the upper portion 40 of the anterior body part relative to the lower portion 42 thereof.

The upper portion bridge 56 carries a circular boss on its upper face, surrounding the bore therein for the guide shaft. The guide includes an anterior stylus 60 which has a slot 62 formed in it, allowing the stylus to be positioned on the upper portion bridge with the boss located in the slot in the stylus. The guide includes a clamp nut 64 which can be tightened against the boss, so as to clamp the stylus against the upper portion bridge. The clamp nut can be loosened to allow the stylus to slide relative to the upper portion bridge. The stylus has markings on it which enable the surgeon to position the stylus correctly relative to the upper portion bridge, according to the size of the femur measured along the anterior-posterior axis and indicated using the scale markings on the limbs of the front plate of the lower portion of the anterior body part.

The clamp nut has a bore 66 extending through it for receiving the guide shaft 60. The clamp nut has a collet portion 68 which includes flexible fingers 70 at its end. The bore 66 in the clamp nut extends through the collet portion. The fingers can be compressed inwardly so as to grip the surface of the guide shaft. This can be relied on to lock the upper portion of the anterior body part against sliding relative to the lower portion.

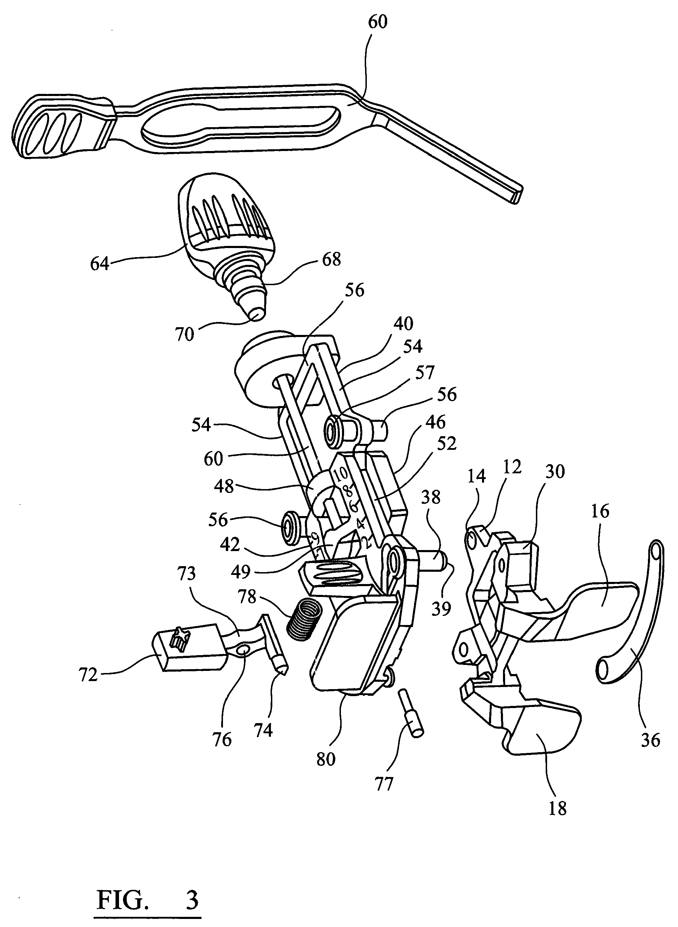

The anterior body part of the guide includes a latching mechanism by which the rotational position of the posterior body part relative to the anterior body part can be locked. The latching mechanism includes a fixed plate 70 and a moving plate 72 which extend from the face of the anterior body part which is opposite to the face which is directed towards the femur when the guide is in use. The moving plate can be seen in Figure 3. It has an arm 73 which extends between the limbs of the front and back plates 44, 46, in a direction towards the posterior body part. The arm has a tooth 74 at its end, the tooth facing towards the moving plate 72. The arm has a bore 76 extending through it. A pivot pin 77 can be inserted through a bore in the anterior body part and the bore in the arm of the moving plate so that the plate can be pivoted about an axis extending through the bore in the arm. A spring 78 provided between the fixed and moving plates of the latch, which urges the plates apart, and urges the tooth 74 to engage the toothed plate 20 on the posterior body part. The application of pressure to the latch so as to move the plates towards one another causes the moving plate to pivot around the pivot pin 77, causing the tooth to become separated from the toothed plate.

The lower portion 42 of the anterior body part of the guide has a ledge 80 extending from it, on the side of the anterior body part which is opposite to that on which the posterior body part of the guide is provided. The underside of the ledge defines a plane which corresponds to the intended plane on which the posterior condyles will be resected. The plane defined by the ledge is approximately parallel to the plane which is defined by the posteriorly extending tab portions 16, 18 on the posterior body part of the guide.

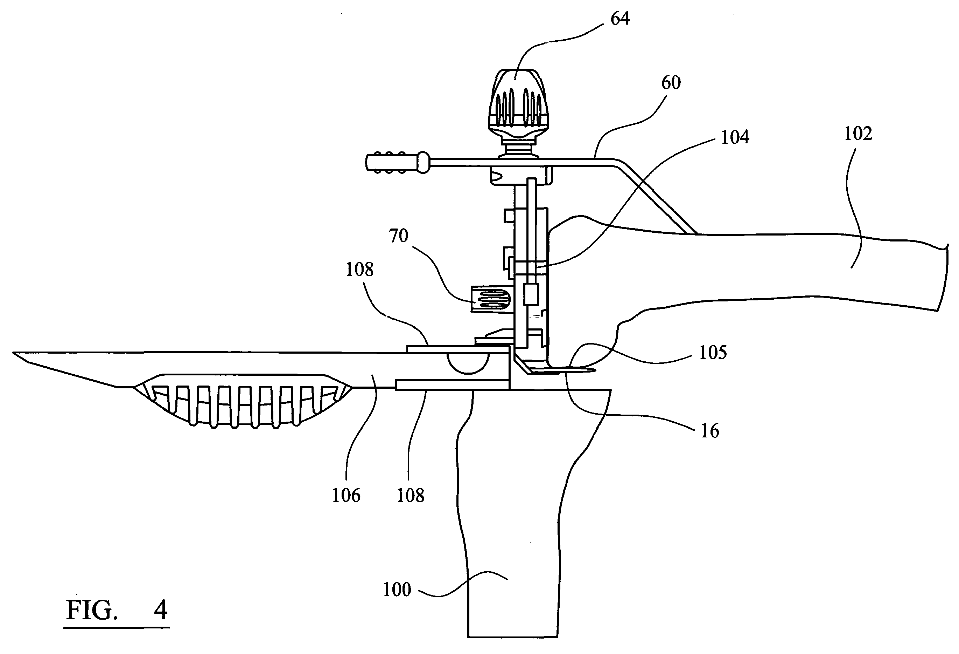

The guide of the invention is used in a surgical procedure to replace a patient's knee joint. It is used after the tibia 100 has been resected proximally, and after the femur 102 has been resected distally, as shown in Figure 4. It is used to measure the size of the femur to provide guidance to the surgeon as to the correct size of femoral component to suit the patient's femur. It is used to place a cutting block on the resected distal face 104 of the femur so that it is appropriately orientated to align the plane of resection of the posterior condyles 105. The guide is used with a spacer 106 which is positioned on the resected tibia as shown in Figure 4, with the underside of the ledge 80 resting on the spacer, the proximal face of the posterior body part in contact with the distal face of the femur, and the posteriorly extending tab portions 16, 18 in contact with the posterior femoral condyles 104. The effective thickness of the spacer can be varied by use of shims 108 such that soft tissue which spans the joint is placed under appropriate tension. The thickness of the spacer corresponds to the thickness of the tibial and bearing component assembly which will be implanted. The selection of tibial and bearing components to ensure appropriate soft tissue tension is something with which orthopaedic surgeons with experience of knee replacement procedures are familiar.

The clamp nut 64 is loosened to allow the stylus 60 to slide relative to the boss on the bridge 56 on the upper portion 40 of the anterior body part 30. Loosening the clamp nut allows the upper portion 40 of the anterior body part to slide relative to the lower portion 42.

The fixed and moving plates 70, 72 of the latch on the anterior body part are squeezed together to release the tooth 74 from between a pair of adjacent teeth on the toothed plate 20 on the posterior body part. This allows the anterior body part to be rotated relative to the posterior body part, about the axis defined by the spigot 34 on the anterior body part and the through-hole 14 on the posterior body part.

Accordingly, the steps of loosening the clamp nut and releasing the latch allow the stylus to be moved relative to the posterior body part until its tip is positioned as desired on the anterior cortex of the patient's femur. The size of the femoral component is indicated by the scale marking on the limbs of the front plate 44 of the lower portion 42 of the anterior body part 30 which the groove in the rim 57 on the limbs of the upper portion of the anterior body part is adjacent to. The angle through which the anterior body part is rotated relative to the posterior body part is selected to provide the appropriate femoral rotation during flexion of the joint. The rotation angle can be selected with reference to anatomical features on the femur such as, for example, one or more of the trochlear and intercondylar grooves and/or the medial/lateral axis. Features (for example fixation holes, shoulders, reference marks, notches, ribs) towards the medial and lateral edges of the posterior body part can be aligned with these and/or other anatomical features to assist alignment of the instrument at a desired rotation angle.

Once the stylus has been positioned as desired on the anterior cortex, the latch is released and the clamp nut is tightened. The guide then defines the positions for fixation pins to be implanted in the distal face of the femur. Those fixation pins can be used in a subsequent step to locate a cutting guide. The cutting guide can be used to guide a saw blade in a step of resecting the patient's femur. The positions for the fixation pins are defined by the posterior pin fixation bores 39 in the spacer lugs 38 (when the surgeon is using a "posterior-up" technique), or by the anterior pin fixation bores 56 in the ends of the limbs of the upper portion 40 of the anterior body part 30 (when the surgeon is using a "anterior-down" technique).

## Claims

1. A guide (2) for locating a plane on which the posterior portions of the femoral condyles are to be resected in a knee replacement procedure and for locating a cutting block on a patient's femur (102), the guide comprising:
a. a posterior body part (4) for positioning against the distal face (104) of the femur, the posterior body part including at least one posteriorly extending tab portion (16,18) for fitting against the posterior condyles,
b. an anterior body part (30) having a proximal face (32) for positioning against the distal face of the femur and an opposite face, the anterior body part being fastened to the posterior body part so that it can rotate relative to the posterior body part about an axis which is approximately perpendicular to the distal face of the femur, and
c. a lock (36) for locking the anterior body part and the posterior body part against relative rotation,
in which the anterior body part has a pair of locator holes (56) formed in it for locating fastener pins by which a cutting block can be fastened to the distal face of the femur, and **characterised in that** the anterior body part has a ledge (80) extending from the said opposite face to indicate the plane of the intended posterior condyle resection plane.

2. A guide (2) as claimed in claim 1, in which the ledge (80) is coincident with the intended resection plane for the posterior condyles (105).

3. A guide (2) as claimed in claim 1, in which the guide includes a stylus (60) for engaging the anterior cortex.

4. A guide (2) as claimed in any one of claims 1 to 3, in which the anterior body part (30) comprises a first portion (42) which is connected to the posterior body part (4), and a second portion (40) on which the stylus (60) mounted, the connection between the first and second portions allowing the second portion to move relative to the first portion, to vary the distance between the posteriorly extending tab portion (16,18) on the posterior body part and the stylus.

5. A guide (2) as claimed in any one of claims 1 to 4, in which the posterior body part (4) includes at least one feature (39) which can be used to fix the posterior body part against movement relative to the femur (102).

6. A kit for use in locating a cutting block on a patient's femur in a knee replacement procedure, the cutting block being used to locate the resection plane for the posterior condyles, which comprises a guide (2) as claimed in any one of claims 1 to 5 and a spacer block (106) for fitting between the ledge (80) and the proximal surface of the tibia (100).

7. A kit for use in locating a cutting block on a patient's femur (102) in a knee replacement procedure, which comprises a guide (2) as claimed in any one of claims 1 to 5 and a cutting block for locating the resection plane for the posterior condyles (105), the cutting block having a pair of fixation holes formed in it for receiving fastener pins for fastening the cutting block to the femur, and having a guide surface for guiding a cutting instrument to cut the posterior condyles, the relative location of the guide surface and the fixation holes on the cutting block being the same as the relative location of the location holes and the posterior facing surface of the ledge on the anterior body part.

## Patentansprüche

1. Führung (2) zum Positionieren einer Ebene, auf der die hinteren Abschnitte der Oberschenkelgelenkknorren in einem Kniegelenkersatzeingriff reseziert werden sollen und zum Positionieren eines Schneidblocks auf einem Oberschenkelknochen (102) eines Patienten, wobei die Führung umfasst:
a. einen hinteren Körperteil (4) zum Positionieren gegen die distale Fläche (104) des Oberschenkelknochens, wobei der hintere Körperteil mindestens einen sich nach hinten erstreckenden Laschenabschnitt (16, 18) zum Anpassen an die hinteren Gelenkknorren umfasst,
b. einen vorderen Körperteil (30), der eine proximale Fläche (32) zum Platzieren gegen die distale Fläche des Oberschenkelknochens und eine gegenüberliegende Fläche aufweist, wobei der vordere Körperteil an dem hinteren Körperteil befestigt ist, sodass er sich relativ zum hinteren Körperteil um eine Achse drehen kann, die annähernd senkrecht zu der distalen Fläche des Oberschenkelknochens ist, und
c. eine Verriegelung (36) zum Verriegeln des vorderen Körperteils und des hinteren Körperteils gegen relative Drehung,
in der der hintere Körperteil ein Paar Positionierlöcher (56) aufweist, die in dem hinteren Körperteil zum Positionieren von Befestigungsstiften gebildet sind, durch die ein Schneidblock an die distale Fläche des Oberschenkelknochens befestigt werden kann, und **dadurch gekennzeichnet, dass** der vordere Körperteil einen Vorsprung (80) aufweist, der sich von der gegenüberliegenden Fläche erstreckt, um die Ebene der beabsichtigten hinteren Gelenkknorrenresektionsebene anzugeben.

2. Führung (2) nach Anspruch 1, wobei der Vorsprung (80) übereinstimmend mit der beabsichtigten Resektionsebene für die hinteren Gelenkknorren (105) ist.

3. Führung (2) nach Anspruch 1, wobei die Führung einen Stylus (60) zum Eingreifen in die vordere Knochenrinde aufweist.

4. Führung (2) nach einem der Ansprüche 1 bis 3, wobei der vordere Körperteil (30) einen ersten Abschnitt (42) umfasst, der mit dem hinteren Körperteil (4) verbunden ist, und einen zweiten Abschnitt (40), an dem der Stylus (60) gelagert ist, wobei die Verbindung zwischen dem ersten und zweiten Abschnitt dem zweiten Abschnitt erlaubt, sich relativ zum ersten Abschnitt zu bewegen, um den Abstand zwischen dem sich nach hinten erstreckenden Laschenabschnitt (16, 18) auf dem hinteren Körperteil und dem Stylus zu variieren.

5. Führung (2) nach einem der Ansprüche 1 bis 4, wobei der hintere Körperteil (4) mindestens ein Merkmal (39) umfasst, das verwendet werden kann, den hinteren Körperteil gegen Bewegung relativ zum Oberschenkel (102) zu fixieren.

6. Kit zur Verwendung für das Positionieren eines Schneidblocks auf einem Oberschenkelknochen eines Patienten bei einem Kniegelenkersatzeingriff, wobei der Schneidblock verwendet wird, die Resektionsebene für die hinteren Gelenkknorren zu positionieren, das eine Führung (2) nach einem der Ansprüche 1 bis 5 und einen Abstandshalter (106) zum Einpassen zwischen den Vorsprung (80) und die proximale Oberfläche des Schienbeins (100) umfasst.

7. Kit zur Verwendung für das Positionieren eines Schneidblocks auf einem Oberschenkelknochen (102) eines Patienten in einem Kniegelenkersatzeingriff, das eine Führung (2) nach einem der Ansprüche 1 bis 5 und einen Schneidblock zum Positionieren der Resektionsebene für die hinteren Gelenkknorren (105) umfasst, wobei der Schneidblock ein Paar Fixierlöcher aufweist, die darin zum Aufnehmen von Befestigungsstiften zum Befestigen des Schneidblocks an den Oberschenkel gebildet sind, und eine Führungsoberfläche zum Führen eines Schneidinstruments zum Schneiden der hinteren Gelenkknorren aufweist, wobei die relative Position der Führungsoberfläche und der Fixierlöcher auf dem Schneidblock übereinstimmt mit der relativen Position der Aufnahmelöcher und der nach hinten zeigenden Oberfläche des Vorsprungs auf dem vorderen Körperteil.

## Revendications

1. Guide (2) pour localiser un plan sur lequel les portions postérieures des condyles fémoraux doivent faire l'objet d'une résection lors d'une procédure de remplacement du genou et pour localiser un bloc de coupe sur le fémur (102) d'un patient, le guide comprenant :
a. une partie de corps postérieure (4) pour un positionnement contre la face distale (104) du fémur, la partie de corps postérieure incluant au moins une portion de patte s'étendant postérieurement (16, 18) pour un ajustement contre les condyles postérieurs,
b. une partie de corps antérieure (30) comportant une face proximale (32) pour un positionnement contre la face distale du fémur et une face opposée, la partie de corps antérieure étant attachée à la partie de corps postérieure de telle sorte qu'elle puisse tourner par rapport à la partie de corps postérieure autour d'un axe qui est approximativement perpendiculaire à la face distale du fémur, et
c. un moyen de blocage (36) pour bloquer la partie de corps antérieure et la partie de corps postérieure à l'encontre d'une rotation relative,
dans lequel la partie de corps antérieure comporte deux trous de moyen de localisation (56) formés en son sein pour localiser des broches de moyen d'attachement au moyen desquelles un bloc de coupe peut être attaché à la face distale du fémur, et **caractérisé en ce que** la partie de corps antérieure comporte une pièce d'appui (80) qui s'étend depuis ladite face opposée afin d'indiquer le plan du plan de résection de condyle postérieur visé.

2. Guide (2) selon la revendication 1, dans lequel la pièce d'appui (80) coïncide avec le plan de résection visé pour les condyles postérieurs (105).

3. Guide (2) selon la revendication 1, dans lequel le guide inclut un stylet (60) pour engager le cortex antérieur.

4. Guide (2) selon l'une quelconque des revendications 1 à 3, dans lequel la partie de corps antérieure (30) comprend une première portion (42) qui est connectée à la partie de corps postérieure (4), et une seconde portion (40) sur laquelle le stylet (60) est monté, la connexion entre les première et seconde portions permettant le déplacement relatif de la seconde portion par rapport à la première portion, afin de faire varier la distance entre la portion de patte s'étendant postérieurement (16, 18) sur la partie de corps postérieure et le stylet.

5. Guide (2) selon l'une quelconque des revendications 1 à 4, dans lequel la partie de corps postérieure (4) inclut au moins une caractéristique (39) qui peut être utilisée pour fixer la partie de corps postérieure à l'encontre d'un déplacement par rapport au fémur (102).

6. Kit pour une utilisation lors de la localisation d'un bloc de coupe sur le fémur d'un patient lors d'une procédure de remplacement du genou, le bloc de coupe étant utilisé afin de localiser le plan de résection pour les condyles postérieurs, lequel comprend un guide (2) comme revendiqué selon l'une quelconque des revendications 1 à 5 et un bloc d'espaceur (106) pour un ajustement entre la pièce d'appui (80) et la surface proximale du tibia (100).

7. Kit pour une utilisation lors de la localisation d'un bloc de coupe sur le fémur d'un patient (102) lors d'une procédure de remplacement du genou, lequel comprend un guide (2) comme revendiqué selon l'une quelconque des revendications 1 à 5 et un bloc de coupe pour localiser le plan de résection pour les condyles postérieurs (105), le bloc de coupe comportant deux trous de fixation formés en son sein pour recevoir des broches de moyen d'attachement pour attacher le bloc de coupe sur le fémur, et comportant une surface de guide pour guider un instrument de coupe pour couper les condyles postérieurs, la localisation relative de la surface de guide et des trous de fixation sur le bloc de coupe étant la même que la localisation relative des trous de moyen de localisation et de la surface faisant face postérieure de la pièce d'appui sur la partie de corps antérieure.
